(19) Europäisches Patentamt
European Patent Office
Office européen des brevets

(11) Publication number: **0 422 591 B1**

(12) # EUROPEAN PATENT SPECIFICATION

(45) Date of publication of patent specification: **08.11.95**

(51) Int. Cl.⁶: **C07C 209/68**, B01J 31/14

(21) Application number: **90119354.0**

(22) Date of filing: **09.10.90**

Divisional application 94107630.9 filed on 09/10/90.

The file contains technical information submitted after the application was filed and not included in this specification

(54) **Ortho-alkylation of aromatic amines.**

(30) Priority: **10.10.89 US 419059**

(43) Date of publication of application:
**17.04.91 Bulletin 91/16**

(45) Publication of the grant of the patent:
**08.11.95 Bulletin 95/45**

(84) Designated Contracting States:
**AT BE CH DE DK ES FR GB GR IT LI LU NL SE**

(56) References cited:
**DE-C- 951 501**
**GB-A- 823 223**
**US-A- 4 760 185**

**Rule 132 declaration**

**Hawley's Condensed Chemical Dictionary, p. 346, 11th Edition**

**Chemtech 1989**

(73) Proprietor: **THE DOW CHEMICAL COMPANY**
**2030 Dow Center,**
**Abbott Road**
**Midland,**
**Michigan 48640 (US)**

(72) Inventor: **Lee, Guo-Shuh John**
**10 Burrell Court**
**Midland,**
**Michigan 48640 (US)**
Inventor: **Durvasula, Rao V.**
**127 Plantation Drive West,**
**Apt. 1504**
**Lake Jackson,**
**Texas 77566 (US)**
Inventor: **Hartwell, Georg E.**
**2908 Georgetown Drive**
**Midland,**
**Michigan 48640 (US)**
Inventor: **Anderson, Kirk D.**
**Route 3,**
**Box 7121**
**Brazoria,**
**Texas 66422 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Inventor: **Moreno, Louis N.**
**61 Greenvale Court**
**Lake Jackson,**
**Texas 77566 (US)**
Inventor: **Shah, Nirad N.**
**120 Dewberry Drive**
**Lake Jackson,**
**Texas 77566 (US)**

(74) Representative: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

## Description

The present invention relates to a process for the alkylation of aromatic amines, particularly the ortho-alkylation of aromatic diamines.

Processes for the preparation of ortho-alkylated aromatic amines from alkenes are known in the art. In one process, aluminum is dissolved in aniline to form aluminum anilide which serves as a catalyst for the alkylation of the aniline or of other aromatic amines. Such a process is taught in U. S. Patent 3,649,693 to Napolitano. Napolitano teaches that the alkylation itself is conducted at significantly elevated temperatures and pressures. Various other references have taught that the addition of mercury chloride, various Friedel Crafts catalysts, iodine or iodine compounds are helpful in improving the efficiency of the reaction.

U. S. Patent 4,760,185 to Becker teaches heating a diamine with an aluminum/zinc alloy and aluminum chloride in the absence of aniline until the evolution of hydrogen is complete. This catalyst mixture is then reacted with a lower alkene at significantly elevated pressure and temperature to form the alkylated phenylenediamine.

DE 951501 teaches the use of aluminum/bronze or Al/Ni in the presence of $HgCl_2$ in a process for the alkylation of aromatic amines with olefins.

The methods known for the ortho-alkylation of aromatic diamines require substantially elevated reaction pressures and temperatures; require the presence of environmentally questionable substances such as mercury chloride; require expensive catalysts or require some combination of these factors. Thus what is needed is a method for the ortho-alkylation of aromatic amines which utilizes less expensive, less toxic catalysts and which utilizes relatively mild alkylation conditions.

The present invention involves a catalyst useful in a process for the ortho-alkylation of aromatic amines wherein the catalyst is prepared by heating an amine with

(1) an aluminum alloy selected from aluminum/copper alloys having a copper content from 0.5 to 30 weight percent and aluminum/boron alloys having a boron content from 0.5 to 30 weight percent;

(2) a Friedel Crafts catalyst selected from aluminum chloride, $SnCl_4$, $SiCl_4$, $ZrI_4$, $FeCl_3$ and $BCl_3$;

(3) optionally, zinc; and

(4) optionally, a solvent.

The completion of catalyst formation is indicated by the completion of the evolution of hydrogen. The present invention also involves the catalyst prepared by this method.

This invention also involves a process for the ortho-alkylation of aromatic amines wherein all or a portion of the catalyst prepared as described above is reacted, optionally with the addition of a second aromatic amine which may be the same as or different than the first aromatic amine, with an alkene at a temperature of at least 250 °C and no greater than 350 °C and a pressure of at least 1,800 kPascals and no greater than 21,600 kPascals under reaction conditions sufficient to produce the ortho-alkylated aromatic amines.

The process of the present invention results in the relatively rapid formation of the ortho-alkylated aromatic amines with high yields and selectivities.

The alkylated amines prepared by the process of this invention have a variety of uses. For example, alkylated phenylenediamines prepared by the process of this invention have a broad range of uses including use as antiknook agents in gasoline; as intermediates in the dye industry and as amine extenders in reaction injection molding.

Aromatic amines useful in the preparation of the catalysts of this invention include those compounds having at least one aromatic ring and at least one amine substituent directly bonded to the aromatic ring or rings.

In a preferred embodiment, aromatic diamines are useful in the preparation of the catalysts of this invention. Such preferred aromatic diamines correspond to the following formulas:

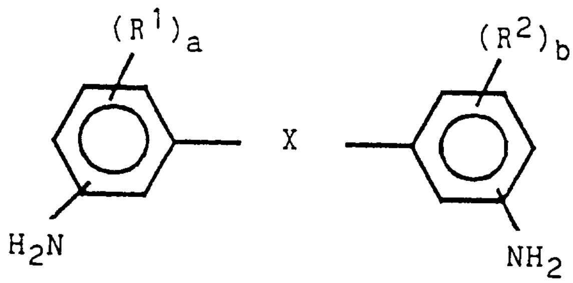

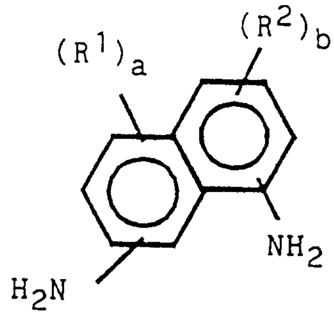

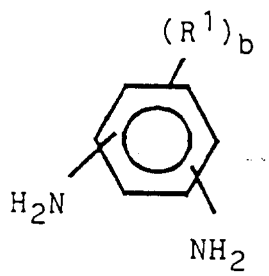

wherein X is a covalent bond, oxygen, sulfur or --CCR$^3$R$^4$)n-- wherein $R^3$ and $R^4$ are separately in each occurrence hydrogen or lower alkyl and n is 1 to 3; $R^1$ and $R^2$ are independently in each occurrence substituted or unsubstituted organic moieties such as alkyl, cycloalkyl and aralkyl moieties; and a and b are independently from zero to three. It is more preferred that $R^1$ and $R^2$ are independently in each occurrence substituted or unsubstituted $C_{1-8}$ organic moieties.

Non-limiting examples of preferred aromatic diamines useful in catalyst preparation include phenylenediamines such as m-phenylenediamine itself or substituted m-phenylenediamines. Useful substituents include $C_{1-8}$ organic moieties such as alkyl, cycloalkyl and aralkyl moieties. It is preferred that the substituents are $C_{1-3}$ alkyl groups. Non-limiting examples of substituted m-phenylenediamines useful in the catalyst preparation by the practice of this invention include 2,4-toluenediamine, 2,6-toluenediamine, 1-ethyl-2,4-phenylenediamine, 1-ethyl-2,6-phenylenediamine, 1-propyl-2,4-phenylenediamine, 1,3-dimethyl-4,6-phenylenediamine, 3,5-diethyl-2,4-toluenediamine, 3,5-diethyl-2,6-toluenediamine, 1-benzyl-2,4-phenylenediamine and mixtures thereof. The use of 2,4-toluenediamine, 2,6-toluenediamine, 3,5-diethyl-2,4-toluenediamine, 3,5-diethyl-2,6-toluenediamine and mixtures thereof is more preferred in the catalyst preparation process.

Aromatic monoamines such as aniline and substituted aniline are also useful in the preparation of the catalysts of the present invention.

Aluminum/copper alloys useful in the preparation of the catalysts useful in the practice of the present invention are those having a copper content of 0.5 weight percent to 30 weight percent and a corresponding aluminum content of 99.5 weight percent to 70 weight percent. It is more preferred that the copper content is 2 weight percent to 10 weight percent. It is preferred to use aluminum/copper alloys commercially available such as Aluminum 2014, 2024, 2036 and 2219. As will be recognized by those skilled in the art, in addition to aluminum and copper, aluminum/copper alloys may contain small amounts of various other metals such as silicon and magnesium.

Aluminum/boron alloys useful in the preparation of the catalysts useful in the practice of the present invention are those having a boron content of 0.5 weight percent to 30 weight percent and a corresponding aluminum content of 99.5 weight percent to 70 weight percent. It is preferred that the boron content is 2 weight percent to 10 weight percent. Aluminum/boron alloys are commercially available and the use of the commercially available alloys is preferred. As will be recognized by those skilled in the art, in addition to aluminum and boron, aluminum/boron alloys may contain small amounts of various other metals.

A Friedel Crafts catalyst, preferably aluminum chloride, is used in the preparation of the catalysts of the present invention. In addition to aluminum chloride, $SnCl_4$, $SiCl_4$, $ZrI_4$, $FeCl_3$ and $BCl_3$ are useful in the practice of this invention. The use of aluminum chloride is preferred. The use of certain other salts, such as zinc chloride, is not preferred in the practice of the present invention.

The amount of aluminum chloride or other salt used in the preparation of the catalyst is that amount which will result in the formation of a catalyst which will result in the production of the desired alkylated amine. When aluminum chloride is used, the amount is preferably that amount of aluminum chloride which will result in a ratio of chlorine to aluminum atoms being in the range of 3:1 to 1:5; preferably 2:1 to 1:2; and most preferably about 1:1.

Zinc is preferably used in the formation of the catalyst in the practice of this invention. Various forms of zinc are useful such as metallic zinc powder and dialkyl zinc. Any size of zinc particle which will function in the formation of the catalysts of this invention is useful. It is preferred that particle size not be too small due to problems associated with handling zinc dust. It is also preferred that particle size not be too large due to the very slow catalyst formation observed using large particles. It is preferred that the zinc be used in a particle size of from 75 microns in diameter to 850 microns in diameter. It is more preferred that the particles have a size range of from 90 to 300 microns in diameter, even more preferably from 100 to 200 microns in diameter and most preferably 125 to 175 microns.

The catalyst preparation of the present invention may be conducted neat or a solvent may be used. In some instances, a solvent/reactant may be used. In other instances, a solvent which is inert to the reaction may be used.

For purposes of the present invention, a solvent/reactant is a liquid aromatic amine capable of forming a catalyst system with aluminum, which also acts as a solvent. For example, in one embodiment of the present invention, the aromatic amine used in the preparation of the catalyst comprises diethyltoluenediamine which is a liquid in which at least some the various components used in catalyst preparation are soluble and thus acts as a solvent. Without wishing to be bound by any theory, it is believed that a portion of the diethyltoluenediamine present also appears to interact in some manner with the aluminum and other metals present to form the catalyst system which is itself soluble in the diethyltoluenediamine. The phrase "catalyst system" is used to describe the catalytic species that forms from the aromatic amine, aluminum and such other metals as may optionally be present. Non-limiting examples of solvent/reactants useful in the preparation of the catalysts of this invention include diethyltoluenediamine and aniline, including substituted anilines.

Inert solvents useful in the process of this invention are those solvents having good solubility for the catalyst system as described above, aluminum chloride and aromatic amines and also having good chemical and thermal stability. Such solvents include aromatic ether solvents which may be exemplified by phenoxy biphenyl and diphenyl ether; and alkyl polyaromatic solvents which may be exemplified terphenyl and diisopropylbiphenyl.

The catalyst is preferably formed by mixing an amine and solvent in a ratio ranging from 1:9 to 9:1 of amine to solvent by weight. It is more preferred that the amine to solvent ratio is greater than about 1:1. Alternatively, the amine is used without a solvent or a solvent/reactant is used. The aluminum alloy is preferably used in an amount such that there are at least 1 part and no greater than 10 parts of aluminum alloy per 100 parts of the amine. It is more preferred that there are 2 to 6 parts by weight of aluminum alloy per 100 parts by weight of diamine. The zinc, when used, is preferably used in an amount to provide at least 0.05 and no greater than 10 parts zinc by weight per 100 parts of amine It is more preferred that there is 0.1 to 2.5 parts by weight of zinc per 100 parts by weight of amine. Aluminum chloride is preferably used in an amount to provide a chlorine to aluminum atom ratio of about 1:1.

The slurry resulting from the above mixture is heated with the evolution of hydrogen until the metals have dissolved. The completion of catalyst formation is indicated by the cessation of hydrogen evolution. After venting the hydrogen, the resulting catalyst is used in the alkylation process in an amount sufficient to provide preferably at least 1 and no greater than 10 weight percent metals based on the weight of the amine to be alkylated. Metals in this context refer to either the aluminum alloy or the mixture of aluminum alloy and zinc metals. It is preferred to use sufficient catalyst to provide at least 2 and no greater than 6 weight percent metals.

The alkylation process of the present invention is useful in the ortho-alkylation of the amine used in the catalyst preparation and in the ortho-alkylation of amines differing from the amine used in the catalyst preparation. In the former situation, catalyst preparation and alkylation may take place in a single reactor without isolation of the catalyst itself, although separate reactors may be used. In the latter situation, the catalyst is prepared in one step and may be stored or transported as necessary and then used in a separate alkylation process. For example, toluenediamine may be used in the preparation of the catalyst and also be the aromatic amine which is alkylated. In contrast, diethyltoluenediamine may be used in the catalyst preparation and toluenediamine used as the aromatic amine to be alkylated.

As discussed above, the aromatic amine to be alkylated may be the same amine used in the preparation of the catalyst or may be a different amine. The aromatic amines which may be alkylated by the process of this invention are the same as those used in catalyst preparation with the exception that the amines to be alkylated are not substituted in the positions ortho to the amine group(s). In a preferred embodiment, toluenediamine is ortho-alkylated to form diethyltoluenediamine.

The alkenes useful in this invention are alkenes containing from two to eighteen carbon atoms. The alkenes may be unsubstituted or may contain inert substituents and may contain single or multiple unsaturations. It is preferred that the alkenes contain from two to twelve carbon atoms and more preferred that they contain from two to six carbon atoms. It is most preferred to use alkenes containing from two to four carbon atoms. Non-limiting examples of alkenes useful in the alkylation reaction of this invention include ethene, propene and butene. It is particularly preferred to use ethene.

One skilled in the art will recognize that both the alkylation reaction and the catalyst preparation and their combination may be conducted in a batch or continuous manner. As discussed above, the catalyst preparation may be conducted separately from the alkylation reaction in that the catalyst is prepared and then stored and/or transported prior to being used in the alkylation reaction. Alternatively, the catalyst may be prepared, but not isolated or collected prior to being used in the alkylation reaction. Those skilled in the art will recognize that choice of reactors will depend in part on whether the catalyst preparation and alkylation reactions take place in the same reactor. The reactor in which the catalyst is prepared must be designed to withstand hydrogen production and should be free of metals known to be detrimental to the formation of the catalyst system. An example of such a reactor is a glass-lined reactor. The reactor used in the alkylation process should be inert and capable of withstanding elevated pressures and temperatures. Examples of reactors useful in the alkylation process include those constructed of carbon steel, titanium and zirconium. Clearly, those processes in which the catalyst is prepared and the alkylation reaction occurs in the same reactor will require a reactor which meets the criteria of both.

The choice of conditions useful in the alkylation reaction of the present invention will depend, in part, on the choice of alkene used as an alkylating agent. For example, one skilled in the art will recognize that alkylation pressures and temperatures will vary depending on the alkylating agent. One skilled in the art will also recognize that the choice of other reaction parameters, whether the alkylation reaction should be done in a batch or a continuous manner and similar decisions will be made based on the particular requirements of a given process. It will also be recognized that one skilled in the art understands the dangers of working at high presssures and that selections of temperatures and pressures will be made in accordance with safe engineering practices.

In this invention temperatures for the alkylation process are at least 250 °C and no greater than 350 °C. If the alkylene used is ethene, it is more preferred that the ethylation process be conducted at temperatures of at least 270 °C and no greater than 330 °C. Ethene pressures preferred in alkylation process range from at least 1800 kPascals up to 21,600 kPascals. Due to the expense of operating high pressure equipment, lower pressures are often preferred for commercial reasons. In a particularly preferred embodiment, the ethylation step is conducted at pressures of at least 1800 kPascals to no greater than 10,800 kPascals, more preferably no greater than 7200 kPascals, and most preferably no greater than 4800 kPascals.

Unless stated otherwise, all parts and percentages are by weight.

Example 1 -- Preparation of Diethyltoluenediamine

A 150-g portion of 2,4-toluenediamine was heated with 5 g of an aluminum/copper alloy containing about 4.4 percent copper and 7.5 g of aluminum chloride to 200 °C with stirring in a 600 cubic centimeter Hastelloy* C Parr (Hastelloy is a trademark of the Cabot Corporation) reactor with a glass liner with a mechanical stirrer. Hydrogen began to evolve at about 175 °C and was complete after about 90 minutes. After venting the hydrogen, the reaction mixture was reacted with ethene in a stirred (1500 rpm) autoclave at a temperature of 290 to 300 °C and an ethene pressure of 7200 kPascals. The alkylation was complete after two hours and the yield of diethyltoluenediamine was 96 percent based on the toluenediamine reacted as determined by gas chromatography.

Example 2

The general procedure outlined in Example 1 was followed. A mixture of 2,4-toluenediamine and 2,6-toluenediamine in a weight ratio of about 80:20 was heated with 3.0 g of aluminum/copper alloy containing about 6.3 percent copper and 4.5 g of aluminum chloride. In Run 1, no zinc powder was added with the aluminum/copper alloy and in Run 2, 1.0 g of zinc powder (average particle diameter of 149 microns, 100 mesh, U.S. Standard) was added with the aluminum/copper alloy. In each case, the reaction was allowed to proceed for one hour. The results obtained are shown in Table I below.

Table I

| Run | Zinc Powder (g) | % Yield of Diethyltoluenediamine |
|---|---|---|
| 1 | 0 | 8 |
| 2 | 1 | 96 |

A comparison of Examples 1 and 2 demonstrates that the addition of zinc to the catalyst preparation results in an alkylation process which gives comparable yields of diethyltoluenediamine in substantially less time and with lower catalyst dosage than those obtained in the absence of the zinc powder.

Example 3

In the following examples, 50 g toluenediamine, 25 g diethylaniline and the amounts of aluminum alloy and aluminum chloride specified in Table II below, were charged to a 300 ml stainless steel Parr reactor flushed with nitrogen. In Runs 1-4, the aluminum/boron alloy used contained about five percent boron. In Runs 5 and 6, the aluminum/copper alloy used contained about six percent copper. In Runs 3 and 4, a glass-lined reactor was used. In the remaining runs, an unlined reactor was used. Also in Run 4, zinc powder (particle diameter of 149 microns, 100 mesh U.S. Standard) was added in the amount specified. The system was closed and heated to about 200 °C for about one hour with stirring. The stirring was stopped and the generated hydrogen vented. The reactor was flushed with nitrogen and then charged with ethene to maintain the pressure specified in the table below. Stirring was resumed and the reactor was heated to 300 °C and maintained at this temperature. The pressure was varied in some of the runs as indicated in the table below. The uptake of ethene was noted and heating was stopped at the time specified in the table below. Ethene was vented out when the reactants were hot. The reactants were cooled to room temperature and the reaction mixture was analyzed by gas chromatography. The results obtained are shown in Table II below.

Table II

| Run | Catalyst | Weight of Catalyst (g) | Solvent | Pressure (kPas) | Time (min) | Conversion | % Yield DETDA |
|---|---|---|---|---|---|---|---|
| 1 | Al-B/$AlCl_3$ | 0.7/1.7 | No | 7200 | 90 | 65 | 24.3 |
| 2 | Al-B/$AlCl_3$ | 0.7/1.7 | DEA | 7200 | 240 | 73.8 | 29.1 |
| 3 | Al-B/$AlCl_3$ | 1.4/3.4 | DEA | 4800 | 150 | 97.8 | 73.7 |
| 4 | Al-B/Zn/$AlCl_3$ | 1.4/1.0/3.4 | DEA | 7200 | 15 | 99.6 | 93.5 |
| 5 | Al-Cu/$AlCl_3$ | 1.6/3.4 | DEA | 7200 | 120 | 78.2 | 30.1 |
| 6 | Al-Cu/$AlCl_3$ | 0.8/1.7 | DEA | 7200 | 270 | 78.5 | 32.4 |

The data in Table II above demonstrate the effectiveness of the present invention.

**Claims**

**Claims for the following Contracting States : DE, GB, FR, IT, NL, SE, LI, CH, BE, AT, LU, GR, DK**

**1.** A method of preparing a catalyst comprising heating an amine with

(1) an aluminum alloy selected from aluminum/copper alloys having a copper content from 0.5 to 30 weight percent and aluminum boron alloys having a boron content from 0.5 to 30 weight percent;

(2) a Friedel Crafts catalyst selected from aluminum chloride, $SnCl_4$, $SiCl_4$, $ZrI_4$, $FeCl_3$ and $BCl_3$;
(3) optionally, zinc; and
(4) optionally, a solvent.

2. The method of Claim 1 wherein the amine is selected from toluenediamine, diethyltoluenediamine and mixtures thereof.

3. The method of Claims 1 or 2 wherein the Friedel Crafts catalyst is aluminum chloride.

4. The method of Claims 1, 2 or 3 wherein zinc is used and has an average particle diameter ranging from 75 microns to 850 microns.

5. A process for the preparation of ortho-alkylated amines comprising preparing a catalyst by a method according to claim 1 and subsequently reacting the catalyst with an alkene and, optionally, a second aromatic amine at a temperature of at least 250 °C and no greater than 350 °C and a pressure of at least 1800 kPascals and no greater than 21,600 kPascals under reaction conditions sufficient to produce the ortho-alkylated amines.

6. The process of Claim 5 wherein the first aromatic amine is selected from the group consisting of toluenediamine, diethyltoluenediamine and mixtures thereof.

7. The process of Claims 5 or 6 wherein the second aromatic amine is used and is toluenediamine.

8. The process of Claims 5, 6 or 7 wherein zinc is used in the preparation of the catalyst and has a particle diameter of from 75 to 850 microns.

9. The process of Claim 8 wherein the elevated pressure is no greater than 7200 kPascals.

10. A catalyst obtainable by heating an amine with
(1) an aluminum alloy selected from aluminum/copper alloys having a copper content from 0.5 to 30 weight percent and aluminum/boron alloys having a boron content from 0.5 to 30 weight percent;
(2) a Friedel Crafts catalyst selected from aluminum chloride, $SnCl_4$, $SiCl_4$, $ZrI_4$, $FeCl_3$ and $BCl_3$;
(3) optionally, zinc; and
(4) optionally, a solvent.

**Claims for the following Contracting State : ES**

1. A method of preparing a catalyst comprising heating an amine with
(1) an aluminum alloy selected from aluminum/copper alloys having a copper content from 0.5 to 30 weight percent and aluminum boron alloys having a boron content from 0.5 to 30 weight percent;
(2) a Friedel Crafts catalyst selected frog aluminum chloride, $SnCl_4$, $SiCl_4$, $ZrI_4$, $FeCl_3$ and $BCl_3$;
(3) optionally, zinc; and
(4) optionally, a solvent.

2. The method of Claim 1 wherein the amino is selected from toluenediamine, diethyltoluenediamine and mixtures thereof.

3. The method of Claims 1 or 2 wherein the Friedel Crafts catalyst is aluminum chloride.

4. The method of Claims 1, 2 or 3 wherein zinc is used and has an average particle diameter ranging from 75 microns to 850 microns.

5. A process for the preparation of ortho-alkylated amines comprising preparing a catalyst by a method according to claim 1 and subsequently reacting the catalyst with an alkene and, optionally, a second aromatic amine at a temperature of at least 250 °C and no greater than 350 °C and a pressure of at least 1800 kPascals and no greater than 21,600 kPascals under reaction conditions sufficient to produce the ortho-alkylated amines.

6. The process of Claim 5 wherein the first aromatic amine is selected from the group consisting of toluenediamine, diethyltoluenediamine and mixtures thereof.

7. The process of Claims 5 or 6 wherein the second aromatic amine is used and is toluenediamine.

8. The process of Claims 5, 6 or 7 wherein zinc is used in the preparation of the catalyst and has a particle diameter of from 75 to 850 microns.

9. The process of Claim 8 wherein the elevated pressure is no greater than 7200 kPascals.

**Patentansprüche**
**Patentansprüche für folgende Vertragsstaaten : DE, GB, FR, IT, NL, SE, LI, CH, BE, AT, LU, GR, DK**

1. Verfahren zur Herstellung eines Katalysators, umfassend das Erhitzen eines Amins mit
   (1) einer Aluminiumlegierung, ausgewählt aus Aluminium/Kupfer Legierungen mit einem Kupfergehalt von 0,5 bis 30 Gew.-% und Aluminium/Bor Legierungen mit einem Borgehalt von 0,5 bis 30 Gew.-%,
   (2) einem Friedel-Crafts Katalysator, ausgewählt aus Aluminiumchlorid, $SnCl_4$, $SiCl_4$, $ZrI_4$, $FeCl_3$ und $BCl_3$,
   (3) gegebenenfalls Zink, und
   (4) gegebenenfalls einem Lösungsmittel.

2. Verfahren nach Anspruch 1, wobei das Amin ausgewählt ist aus Toluoldiamin, Diethyltoluoldiamin und Gemischen davon.

3. Verfahren nach Anspruch 1 oder 2, wobei der Friedel-Crafts Katalysator Aluminiumchlorid ist.

4. Verfahren nach Anspruch 1, 2 oder 3, wobei Zink verwendet wird und einen durchschnittlichen Teilchendurchmesser im Bereich von 75 bis 850 Mikrometer hat.

5. Verfahren zur Herstellung ortho-alkylierter Amine, umfassend das Herstellen eines Katalysators durch ein Verfahren gemäß Anspruch 1 und anschließend das Umsetzen des Katalysators mit einem Alken und gegebenenfalls mit einem zweiten aromatischen Amin bei einer Temperatur von 250°C und nicht mehr als 350°C und einem Druck von 1800 kPascal und nicht mehr als 21.600 kPascal unter ausreichenden Reaktionsbedingungen um die ortho-alkylierten Amine herzustellen.

6. Verfahren nach Anspruch 5, wobei das erste aromatische Amin ausgewählt wird aus der Gruppe bestehend aus Toluoldiamin, Diethyltoluoldiamin und Gemischen davon.

7. Verfahren nach Anspruch 5 oder 6, wobei das zweite aromatische Amin verwendet wird und Toluoldiamin ist.

8. Verfahren nach Anspruch 5, 6 oder 7, wobei Zink bei der Katalysatorherstellung verwendet wird und einen Teilchendurchmesser im Bereich von 75 bis 850 Mikrometer hat.

9. Verfahren nach Anspruch 8, wobei der erhöhte Druck nicht mehr als 7200 kPascal beträgt.

10. Katalysator, erhältlich durch Erhitzen eines Amins mit
   (1) einer Aluminiumlegierung, ausgewählt aus Aluminium/Kupfer Legierungen mit einem Kupfergehalt von 0,5 bis 30 Gew.-% und Aluminium/Bor Legierungen mit einem Borgehalt von 0,5 bis 30 Gew.-%,
   (2) einem Friedel-Crafts Katalysator, ausgewählt aus Aluminiumchlorid, $SnCl_4$, $SiCl_4$, $ZrI_4$, $FeCl_3$ und $BCl_3$,
   (3) gegebenenfalls Zink, und
   (4) gegebenenfalls einem Lösungsmittel.

**Patentansprüche für folgenden Vertragsstaat : ES**

1. Verfahren zur Herstellung eines Katalysators, umfassend das Erhitzen eines Amins mit
   (1) einer Aluminiumlegierung, ausgewählt aus Aluminium/Kupfer Legierungen mit einem Kupfergehalt von 0,5 bis 30 Gew.-% und Aluminium/Bor Legierungen mit einem Borgehalt von 0,5 bis 30 Gew.-%,
   (2) einem Friedel-Crafts Katalysator, ausgewählt aus Aluminiumchlorid, $SnCl_4$, $SiCl_4$, $ZrI_4$, $FeCl_3$ und $BCl_3$,
   (3) gegebenenfalls Zink, und
   (4) gegebenenfalls einem Lösungsmittel.

2. Verfahren nach Anspruch 1, wobei das Amin ausgewählt ist aus Toluoldiamin, Diethyltoluoldiamin und Gemischen davon.

3. Verfahren nach Anspruch 1 oder 2, wobei der Friedel-Crafts Katalysator Aluminiumchlorid ist.

4. Verfahren nach Anspruch 1, 2 oder 3, wobei Zink verwendet wird und einen durchschnittlichen Teilchendurchmesser im Bereich von 75 bis 850 Mikrometer hat.

5. Verfahren zur Herstellung ortho-alkylierter Amine, umfassend das Herstellen eines Katalysators durch ein Verfahren gemäß Anspruch 1 und anschließend das Umsetzen des Katalysators mit einem Alken und gegebenenfalls mit einem zweiten aromatischen Amin bei einer Temperatur von 250 °C und nicht mehr als 350 °C und einem Druck von 1800 kPascal und nicht mehr als 21.600 kPascal unter ausreichenden Reaktionsbedingungen um die orthoalkylierten Amine herzustellen.

6. Verfahren nach Anspruch 5, wobei das erste aromatische Amin ausgewählt wird aus der Gruppe bestehend aus Toluoldiamin, Diethyltoluoldiamin und Gemischen davon.

7. Verfahren nach Anspruch 5 oder 6, wobei das zweite aromatische Amin verwendet wird und Toluoldiamin ist.

8. Verfahren nach Anspruch 5, 6 oder 7, wobei Zink bei der Katalysatorherstellung verwendet wird und einen Teilchendurchmesser im Bereich von 75 bis 850 Mikrometer hat.

9. Verfahren nach Anspruch 8, wobei der erhöhte Druck nicht mehr als 7200 kPascal beträgt.

**Revendications**
**Revendications pour les Etats contractants suivants : DE, GB, FR, IT, NL, SE, LI, CH, BE, AT, LU, GR, DK**

1. Procédé de préparation d'un catalyseur, consistant à chauffer une amine avec
   (1) un alliage d'aluminium choisi parmi les alliages aluminium/cuivre ayant une teneur en cuivre comprise entre 0,5 et 30 % en poids et les alliages aluminium/bore ayant une teneur en bore comprise entre 0,5 et 30 % en poids ;
   (2) un catalyseur de Friedel-Crafts choisi parmi le chlorure d'aluminium, $SnCl_4$, $SiCl_4$, $ZrI_4$, $FeCl_3$ et $BCl_3$ ;
   (3) éventuellement, du zinc ; et
   (4) éventuellement, un solvant.

2. Procédé selon la revendication 1, dans lequel l'amine est choisie parmi la toluènediamine, la diéthyltoluènediamine et leurs mélanges.

3. Procédé selon les revendications 1 ou 2, dans lequel le catalyseur de Friedel-Crafts est le chlorure d'aluminium.

4. Procédé selon les revendications 1, 2 ou 3, dans lequel on utilise du zinc, qui a un diamètre moyen de particules compris entre 75 $\mu$m et 850 $\mu$m.

5. Procedé pour la préparation d'amines ortho-alkylées, qui consiste à préparer un catalyseur par un procédé selon la revendication 1 et ensuite à faire réagir le catalyseur avec un alcène et, éventuellement, une deuxième amine aromatique à une température comprise entre 250 °C et 350 °C, et sous une pression comprise entre 1 800 kPa et 21 600 kPa, dans des conditions de réaction suffisantes pour produire les amines aromatiques ortho-alkylées.

6. Procédé selon la revendication 5, dans lequel la première amine aromatique est choisie dans l'ensemble constitué par la toluènediamine, la diéthyltoluènediamine et leurs mélanges.

7. Procédé selon les revendications 5 ou 6, dans lequel on utilise la deuxième amine aromatique, qui est la toluènediamine.

8. Procédé selon les revendications 5, 6 ou 7, dans lequel le zinc est utilisé dans la préparation du catalyseur et a un diamètre de particules compris entre 75 et 850 µm.

9. Procédé selon la revendication 8, dans lequel la pression élevée n'est pas supérieure à 7 200 kPa.

10. Catalyseur susceptible d'être obtenu en chauffant une amine avec
(1) un alliage d'aluminium choisi parmi les alliages aluminium/cuivre ayant une teneur en cuivre comprise entre 0,5 et 30 % en poids et les alliages aluminium/bore ayant une teneur en bore comprise entre 0,5 et 30 % en poids ;
(2) un catalyseur de Friedel-Crafts choisi parmi le chlorure d'aluminium, $SnCl_4$, $SiCl_4$, $ZrI_4$, $FeCl_3$ et $BCl_3$ ;
(3) éventuellement, du zinc ; et
(4) éventuellement, un solvant.

**Revendications pour les l'Etat contractant suivant : ES**

1. Procédé de préparation d'un catalyseur, consistant à chauffer une amine avec
(1) un alliage d'aluminium choisi parmi les alliages aluminium/cuivre ayant une teneur en cuivre comprise entre 0,5 et 30 % en poids et les alliages aluminium/bore ayant une teneur en bore comprise entre 0,5 et 30 % en poids ;
(2) un catalyseur de Friedel-Crafts choisi parmi le chlorure d'aluminium, $SnCl_4$, $SiCl_4$, $ZrI_4$, $FeCl_3$ et $BCl_3$ ;
(3) éventuellement, du zinc ; et
(4) éventuellement, un solvant.

2. Procédé selon la revendication 1, dans lequel l'amine est choisie parmi la toluènediamine, la diéthyltoluènediamine et leurs mélanges.

3. Procédé selon les revendications 1 ou 2, dans lequel le catalyseur de Friedel-Crafts est le chlorure d'aluminium.

4. Procédé selon les revendications 1, 2 ou 3, dans lequel on utilise du zinc, qui a un diamètre moyen de particules compris entre 75 µm et 850 µm.

5. Procédé pour la préparation d'amines ortho-alkylées, qui consiste à préparer un catalyseur par un procédé selon la revendication 1 et ensuite à faire réagir le catalyseur avec un alcène et, éventuellement, une deuxième amine aromatique à une température comprise entre 250 °C et 350 °C, et sous une pression comprise entre 1 800 kPa et 21 600 kPa, dans des conditions de réaction suffisantes pour produire les amines aromatiques ortho-alkylées.

6. Procédé selon la revendication 5, dans lequel la première amine aromatique est choisie dans l'ensemble constitué par la toluènediamine, la diéthyltoluènediamine et leurs mélanges.

7. Procédé selon les revendications 5 ou 6, dans lequel on utilise la deuxième amine aromatique, qui est la toluènediamine.

8. Procédé selon les revendications 5, 6 ou 7, dans lequel le zinc est utilisé dans la préparation du catalyseur et a un diamètre de particules compris entre 75 et 850 µm.

9. Procédé selon la revendication 8, dans lequel la pression élevée n'est pas supérieure à 7 200 kPa.